# FASCICULE DE BREVET EUROPEEN

(11) **EP 2 595 697 B1**
(45) Date de publication et mention de la délivrance du brevet: **28.12.2016**
(21) Numéro de dépôt: 11744037.0
(22) Date de dépôt: 30.06.2011
(51) Int. Cl.: A61N 1/05, A61N 1/36, C23C 16/04

(54) **PROCÉDÉ DE FABRICATION D'UNE ÉLECTRODE À USAGE MÉDICAL, ET ÉLECTRODE OBTENUE**
VERFAHREN ZUR HERSTELLUNG EINER ELEKTRODE FÜR MEDIZINISCHE ZWECKE UND AUF DIESE WEISE HERGESTELLTE ELEKTRODE
METHOD FOR MANUFACTURING AN ELECTRODE FOR MEDICAL USE, AND ELECTRODE OBTAINED

(30) Priorité: 22.07.2010 FR 1056014
(43) Date de publication de la demande: 29.05.2013
(73) Titulaire: Dixi Microtechniques S.A.S., 25025 Besançon (FR)
(72) Inventeur: DARNIS, Jean-Pierre, F-70700 Cugney (FR)
(74) Mandataire: Koelbel, Caroline
(86) Numéro de dépôt international: PCT/FR2011/000383
(87) Numéro de publication internationale: WO 2012/010747

(56) Documents cités:
- WO-A1-2010/055421
- US-A1- 2005 171 587
- US-A1- 2007 005 112
- US-A1- 2007 007 240
- US-A1- 2009 240 314

## Description

### Domaine technique :

La présente invention concerne un procédé de fabrication d'une électrode à usage médical, telle qu'une électrode intracérébrale destinée à être utilisée au niveau du cerveau, cette électrode se présentant sous la forme d'une tige étroite et allongée portant au moins un plot de contact électrique relié à un conducteur électrique destiné à être raccordé à un appareil de traitement et/ou d'enregistrement.

La présente invention concerne également une électrode obtenue par ledit procédé.

### Technique antérieure :

La présente invention concerne plus particulièrement le domaine des électrodes intracérébrales. De tels instruments sont utilisés pour réaliser des explorations électrophysiologiques cérébrales, notamment en vue de localiser et caractériser des foyers épileptogènes de patients épileptiques, diagnostiquer et/ou traiter des tumeurs, ou recueillir des activités spontanées. Les électrodes intracérébrales permettent également d'effectuer des stimulations telles que du mapping fonctionnel, ou déclencher des crises. Une autre application concerne encore la réalisation de thermocoagulations destinées au traitement de l'épilepsie, à l'issue d'une séance de stimulation stéréoélectroencéphalographique.

Les électrodes intracérébrales sont classiquement composées de contacts électriques, d'isolants, de conducteurs, d'une gaine et de connecteurs assemblés entre eux. Du fait des très faibles dimensions de ces différentes pièces, leur assemblage est particulièrement fastidieux, requière un temps très long, et génère par conséquent des coûts de main d'oeuvre importants. En outre les électrodes intracérébrales ainsi fabriquées ne donnent à l'heure actuelle pas entière satisfaction du point de vue de leurs qualités intrinsèques. Il a en effet été constaté que la résistance mécanique d'un tel assemblage de pièces hétérogènes de petites dimensions est relativement faible et peut être parfois insuffisante. Un procédé de fabrication d'une électrode de ce type est décrit dans le WO 2010/055421.

D'autre part, un autre inconvénient réside dans le fait que les masses métalliques des contacts électriques sont importantes et engendrent des artéfacts pouvant nuire à la qualité des images des zones explorées ou stimulées, recueillies par imagerie par résonance magnétique (IRM).

### Exposé de l'invention:

La présente invention vise à pallier ces inconvénients en proposant un procédé de fabrication d'une électrode intracérébrale permettant à la fois de diminuer les temps de fabrication, et de fournir une électrode intracérébrale présentant une résistance mécanique accrue avec des masses métalliques réduites.

Dans ce but, l'invention concerne un procédé du genre indiqué en préambule, caractérisé en ce que pour réaliser ladite électrode on utilise un substrat de forme cylindrique et on dépose une couche de métal sur la surface dudit substrat par une technique de dépôt physique en phase vapeur à travers un masque déterminant un motif agencé pour définir au moins ledit plot de contact électrique.

Selon une variante de ce procédé, l'on effectue ledit dépôt métallique en au moins deux étapes successives et l'on retourne ledit substrat entre lesdites deux étapes.

Selon une autre variante du présent procédé, l'on effectue le dépôt métallique en une seule étape au moyen d'au moins deux cibles.

Selon une troisième variante de mise en oeuvre de ce procédé, l'on effectue ledit dépôt métallique en soumettant ledit substrat à une rotation.

Selon une autre caractéristique du présent procédé, l'on utilise un masque en acier inoxydable formant une pince agencée pour enserrer ledit substrat.

Une caractéristique additionnelle est encore définie par le fait que l'on active chimiquement la zone du substrat non recouverte par ledit masque, de préférence en le soumettant à une étape de nettoyage ionique effectué au moyen d'un mélange d'oxygène et d'argon à l'état de plasma, puis en y déposant une couche de titane. Avantageusement, l'on utilise en tant que métal pour définir ledit plot de contact électrique, de l'or.

L'invention a également pour objet une électrode à usage médical, obtenue par la mise en oeuvre du procédé précédemment décrit, telle qu'une électrode intracérébrale destinée à être utilisée au niveau du cerveau, cette électrode se présentant sous la forme d'une tige étroite et allongée comportant au moins un plot de contact électrique relié à un conducteur électrique destiné à être raccordé à un appareil de traitement et/ou d'enregistrement, caractérisée en ce que ladite tige constitue un substrat cylindrique autour duquel est déposée au moins une couche de métal par une technique de dépôt physique en phase vapeur à travers un masque déterminant un motif agencé pour définir au moins ledit plot de contact électrique. De préférence, ledit plot de contact électrique est réalisé en or.

### Description sommaire des dessins :

La présente invention et ses avantages apparaîtront mieux dans la description suivante d'un mode de réalisation donné à titre d'exemple non limitatif, en référence aux dessins annexés, dans lesquels:
- la figure 1 représente une vue en coupe d'un dispositif pour la mise en oeuvre du procédé selon l'invention, agencé pour effectuer le dépôt métallique en soumettant le substrat à une rotation,
- la figure 2 est une vue de dessus d'une électrode selon l'invention dont une extrémité comporte des câbles de raccordement agencés pour être raccordés à un connecteur,
- la figure 3 est une vue de dessus d'une variante de l'électrode selon l'invention dans laquelle le connecteur est intégré,
- la figure 4 est vue en perspective d'un masque utilisé dans le cadre de la mise en oeuvre du procédé selon l'invention, en position ouverte, et
- la figure 5 est une vue de côté du masque de la figure 4, en position fermée.

### Illustrations de l'invention et meilleure manière de la réaliser:

En référence aux figures, la présente invention concerne un procédé de fabrication d'une électrode intracérébrale 1, de forme cylindrique comportant de manière classique, une pluralité de plots de contact électrique 2, répartis sur le pourtour et sur au moins une partie de sa longueur l. Ces plots de contact électrique 2 permettent d'effectuer les opérations telles que, par exemple, l'enregistrement de l'activité cérébrale, la stimulation cérébrale et/ou la réalisation de thermolésions précédemment évoquées.

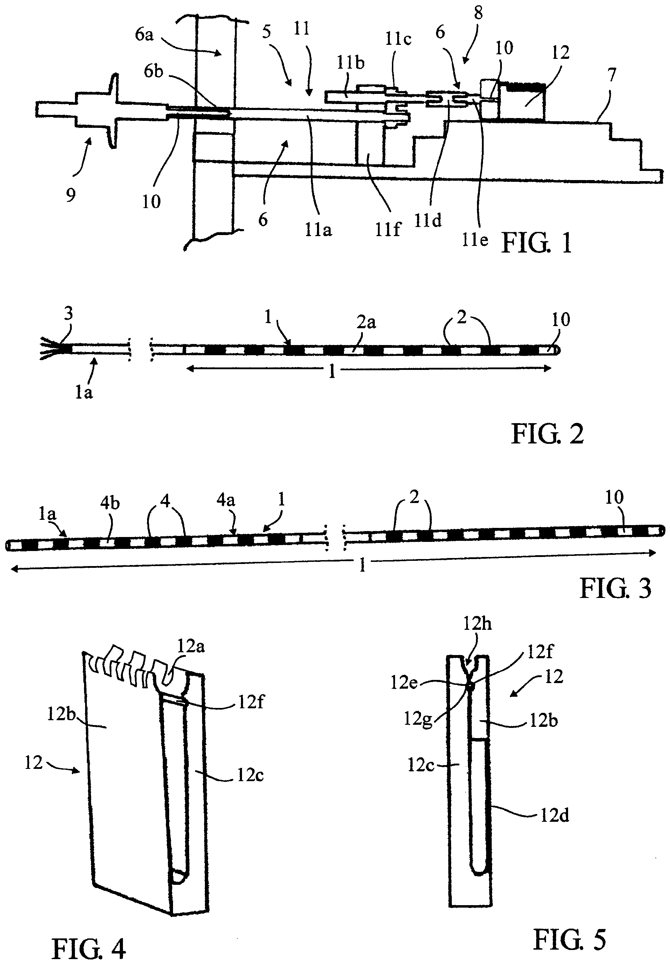

Selon une variante de réalisation représentée à la figure 2, les plots de contact 2 peuvent être reliés à une pluralité de câbles de connexion 3 prolongeant une extrémité la de l'électrode 1 et agencés pour raccorder ladite électrode 1 au travers d'un connecteur non représenté, par exemple de type multicontact, sur l'équipement médical choisi en fonction de la nature des opérations à effectuer au niveau du cerveau. Selon une autre variante, représentée à la figure 3, l'électrode 1 comporte avantageusement, au niveau de son extrémité la, une série de plots de contact 4 reliés électriquement aux plots de contact 2 de l'électrode 1 et définissant un connecteur multicontact cylindrique 4a intégré, agencé pour être raccordé directement audit équipement médical.

Une telle électrode intracérébrale 1 présente classiquement une structure rigide ou semi-rigide, d'un diamètre d'environ 0,8mm, tandis que sa longueur l, et par conséquent le nombre de plots de contact 2, peuvent être variables.

Dans l'invention représenté, l'électrode intracérébrale 1 est fabriquée à partir d'un substrat 10, de forme cylindrique, qui définit le corps de l'électrode, sur la surface duquel est déposée, par une technique de dépôt physique en phase vapeur, une couche de métal, de préférence de l'or, ou tout métal électriquement conducteur équivalent, selon un motif définissant les plots de contact 2, et le cas échéant les plots de contact 4. Le substrat 10 employé est réalisé à partir d'un matériau biocompatible, adapté à une implantation dans le tissu cérébral, ce matériau étant par exemple choisi dans le groupe comprenant le polyamide, la polyétheréthercétone, le pebax®, le polyimide, le polyimide renforcé, le polyuréthane, le poyuréthane Tecoflex®, le Rislan®.

Afin de garantir un dépôt de métal uniforme sur l'ensemble de la circonférence du substrat 10, le présent procédé prévoit avantageusement, conformément à la variante de réalisation illustrée, de soumettre celui-ci à une rotation durant toutes les étapes de fabrication de l'électrode 1. Ce but est atteint par la mise en oeuvre du dispositif 5, comportant de manière conventionnelle une enceinte à vide 6 équipée d'une cuve 7 de dépôt physique en phase vapeur, et des moyens de réception 8 du substrat 10 agencés pour soumettre celui-ci à une rotation au sein de ladite enceinte à vide 6. Lesdits moyens de rotation 8 sont commandés à partir de l'espace extérieur à ladite enceinte 6 et sont couplés à cet effet à des moyens d'entraînement 9, tels qu'un moteur électrique ou similaire (non représenté), situés à l'extérieur de ladite enceinte à vide 6, au moyen d'un arbre moteur 11 traversant une paroi 6a de l'enceinte à vide 6 par un palier étanche 6b. L'arbre moteur 11 peut être couplé directement ou indirectement aux moyens de réception 8 comme dans l'exemple représenté. A cet effet, l'arbre moteur 11 comporte un premier axe 11a couplé d'une part au moteur et d'autre part à un second axe 11b au travers d'un engrenage comportant deux roues dentées 11c. L'axe 11b est lui-même couplé, au travers d'un support isolant 11d, à un troisième axe 11e relié à une extrémité du substrat 10. Les axes 11a, 11b sont de préférence guidés dans des paliers prévus dans un support 11f garantissant leur positionnement, parallélisme et alignement.

Conformément au présent procédé, le dépôt de la couche de métal définissant les plots de contact 2, 4 est effectué après une étape d'activation chimique de la zone dudit substrat 10 concernée. Cette étape d'activation, réalisée par exemple au sein du dispositif 5, consiste à réaliser un nettoyage ionique de ladite zone concernée du substrat 10, par exemple au moyen d'un mélange d'oxygène et d'argon suivi du dépôt d'une couche de titane.

Avantageusement, les étapes d'activation du substrat 10, puis de dépôt de la couche métallique, sont réalisées en enfermant l'extrémité libre du substrat 10 à l'intérieur d'un masque 12 présentant des ouvertures 12a déterminant le motif agencé pour définir les plots de contact électrique 2, 4. Selon un exemple de réalisation illustré par les figures 4 et 5, le masque 12 peut se présenter sous la forme d'une pince comportant deux branches parallèles 12b, 12c, reliées à leur base par une languette élastique 12d qui sollicite lesdites branches 12b, 12c en position fermée. Un tel masque 12 comporte un logement de guidage 12e (cf. fig.5) apte à recevoir le substrat 10, ce logement étant issu du rapprochement entre elles de deux rainures 12f, 12g formées sur les faces internes des branches 12b, 12c du masque 12. Les extrémités libres des branches 12b, 12c forment une zone d'introduction 12h en « V » située au dessus du logement de guidage 12e pour permettre la mise en place du substrat 10 par l'ouverture et la fermeture automatique de la pince 12 obtenues grâce à la forme en V de la zone d'introduction et à la languette élastique 12d. Le logement de guidage 12e permet de préserver parfaitement la position du substrat 10 par rapport aux ouvertures 12a pendant les étapes d'activation puis de dépôt de métal. L'utilisation d'un tel masque 12 permet par conséquent de réaliser le dépôt de métal de manière très précise, en évitant tout dépôt intempestif dans les espaces 2a, 4b séparant deux plots de contact 2, 4. De préférence, un tel masque 12 est réalisé par usinage d'une pièce en acier inoxydable, ou tout autre matériau présentant les mêmes propriétés de flexibilité, au moyen d'un procédé de découpe par électroérosion ou par tout autre procédé de fabrication équivalent.

Selon une autre variante du présent procédé, mise en oeuvre au moyen d'un dispositif non représenté, il est également possible d'effectuer le dépôt de métal sur la surface d'un substrat 10 en au moins deux étapes successives. Dans ce cas, il est prévu dans une première étape d'exposer d'abord une première face du substrat 10 audit métal, puis de retourner ledit substrat 10 et de répéter cette même opération sur une autre face dudit substrat 10.

Selon une troisième variante du présent procédé, il est encore possible de procéder audit dépôt de métal simultanément sur au moins deux faces du substrat 10, au moyen d'un dispositif non représenté comportant au moins deux cibles.

Le choix de ces différents procédés sera déterminé en fonction des contraintes de fabrication en série plus ou moins importante de ce type d'électrodes.

### Possibilités d'application industrielle :

Il ressort clairement de cette description que l'invention permet de fabriquer des électrodes intracérébrales de manière automatisée, de grande précision, en série, avec une excellente reproductibilité, permettant de réduire les coûts de main d'oeuvre. Par ailleurs, les tests effectués ont permis de montrer que la couche d'or ainsi déposée présentait une excellente résistance à l'arrachage, conduisant à des électrodes intracérébrales se caractérisant par une résistance mécanique supérieure à celle observée pour les électrodes intracérébrales classiques.

La présente invention n'est pas limitée à l'exemple de réalisation décrit mais s'étend à toute modification et variante évidentes pour un homme du métier tout en restant dans l'étendue de la protection définie dans les revendications annexées.

## Revendications

1. Procédé de fabrication d'une électrode à usage médical, telle qu'une électrode intracérébrale (1) destinée à être utilisée au niveau du cerveau, cette électrode se présentant sous la forme d'une tige étroite, cylindrique et allongée comportant au moins un plot de contact électrique (2, 4) destiné à être relié à un conducteur électrique destiné à être raccordé à un appareil de traitement et/ou d'enregistrement, où pour réaliser ladite électrode (1), on utilise un substrat (10) de forme cylindrique qui définit le corps de l'électrode, réalisé à partir d'un matériau rigide ou semi-rigide biocompatible, **caractérisé en ce qu'**on dépose une couche de métal sur la surface dudit substrat cylindrique (10) par une technique de dépôt physique en phase vapeur à travers un masque (12) déterminant un motif agencé pour définir au moins ledit plot de contact électrique (2, 4).

2. Procédé selon la revendication 1, **caractérisé en ce que** l'on dépose ladite couche de métal sur l'ensemble de la circonférence dudit substrat (10), **en ce que** l'on effectue ledit dépôt métallique en au moins deux étapes successives et **en ce que** l'on retourne ledit substrat (10) entre lesdites deux étapes successives.

3. Procédé selon la revendication 1, **caractérisé en ce que** l'on dépose ladite couche de métal sur l'ensemble de la circonférence dudit substrat (10) et **en ce que** l'on effectue ledit dépôt métallique en une seule étape au moyen d'au moins deux cibles.

4. Procédé selon la revendication 1, **caractérisé en ce que** l'on dépose ladite couche de métal sur l'ensemble de la circonférence dudit substrat (10) et **en ce que** l'on effectue ledit dépôt métallique en soumettant ledit substrat (10) à une rotation.

5. Procédé selon l'une quelconque des revendications précédentes, **caractérisé en ce que** l'on utilise un masque (12) en acier inoxydable formant une pince agencée pour enserrer ledit substrat (10).

6. Procédé selon la revendication 1, **caractérisé en ce que**, préalablement au dépôt de la couche de métal, l'on active chimiquement la zone du substrat (10) non recouverte par ledit masque (12) par un nettoyage ionique effectué au moyen d'un mélange d'oxygène et d'argon à l'état de plasma, puis on y dépose une couche de titane.

7. Procédé selon la revendication 1, **caractérisé en ce que** l'on utilise en tant que métal pour définir ledit plot de contact électrique (2, 4) de l'or.

8. Électrode à usage médical, obtenue par la mise en oeuvre du procédé selon l'une quelconque des revendications 1 à 7, telle qu'une électrode intracérébrale (1) destinée à être utilisée au niveau du cerveau, cette électrode (1) se présentant sous la forme d'une tige étroite, cylindrique et allongée comportant au moins un plot de contact électrique (2, 4) destiné à être relié à un conducteur électrique destiné à être raccordé à un appareil de traitement et/ou d'enregistrement, où ladite tige constitue un substrat (10) cylindrique réalisé à partir d'un matériau rigide ou semi-rigide biocompatible **caractérisée en ce qu'**autour duquel est déposée au moins une couche de métal par une technique de dépôt physique en phase vapeur à travers un masque (12) déterminant un motif agencé pour définir au moins ledit plot de contact électrique (2, 4).

9. Électrode selon la revendication 8, **caractérisée en ce que** ledit plot de contact électrique (2, 4) est réalisé en or.

## Patentansprüche

1. Verfahren zur Herstellung einer Elektrode für medizinische Zwecke wie eine für eine Benutzung im Gehirn bestimmte intrazerebrale Elektrode (1), wobei diese Elektrode die Form eines schmalen zylindrischen und länglichen Stifts mit zumindest einer elektrischen Kontaktfläche (2, 4) aufweist, die dazu bestimmt ist, mit einem elektrischen Leiter verbunden zu werden, der an ein Verarbeitungs- oder Aufnahmegerät angeschlossen wird, bei dem, um besagte Elektrode (1) herzustellen, man ein aus einem biokompatiblen starren oder halbstarren Material hergestelltes Substrat (10) mit einer zylindrischen Form verwendet, das den Körper der Elektrode bildet, **dadurch gekennzeichnet, dass** man mit einem physikalischen Dampfniederschlagsprozess durch eine Maske (12) hindurch, die ein Muster bestimmt, das ausgelegt ist, um zumindest besagte elektrische Kontaktfläche (2, 4) zu definieren, eine Metallschicht auf der Oberfläche von besagtem Substrat (10) abscheidet.

2. Verfahren nach Anspruch 1, **dadurch gekennzeichnet, dass** man besagte Metallschicht auf dem gesamten Umfang von besagtem Substrat (10) abscheidet, dadurch, dass man besagte Metallabscheidung in zumindest zwei aufeinander folgenden Schritten durchführt, und dadurch, dass man besagtes Substrat (10) zwischen besagten zwei aufeinander folgenden Schritten umdreht.

3. Verfahren nach Anspruch 1, **dadurch gekennzeichnet, dass** man besagte Metallschicht auf dem gesamten Umfang von besagtem Substrat (10) abscheidet und dass man besagte Metallabscheidung in einem einzigen Schritt mittels zumindest zweier Targets durchführt.

4. Verfahren nach Anspruch 1, **dadurch gekennzeichnet, dass** man besagte Metallschicht auf dem gesamten Umfang von besagtem Substrat (10) abscheidet und dass man während besagter Metallabscheidung besagtes Substrat (10) rotiert.

5. Verfahren nach einem beliebigen der vorhergehenden Ansprüche, **dadurch gekennzeichnet, dass** man eine Maske (12) aus rostfreiem Stahl verwendet, die eine Zange bildet, die ausgelegt ist, um besagtes Substrat (10) zu umfassen.

6. Verfahren nach Anspruch 1, **dadurch gekennzeichnet, dass**, vor dem Abscheiden der Metallschicht, man den nicht von besagter Maske (12) bedeckten Bereich des Substrats (10) durch eine ionische Reinigung mit einer Sauerstoff- und Argon-Mischung in Plasmazustand chemisch aktiviert und man anschließend eine Schicht Titan darauf abscheidet.

7. Verfahren nach Anspruch 1, **dadurch gekennzeichnet, dass** man als Metall, um besagte elektrische Kontaktfläche (2, 4) zu definieren, Gold verwendet.

8. Elektrode für medizinische Zwecke, erhalten durch Umsetzung des Verfahrens nach einem Beliebigen der Ansprüche 1 bis 7, wie eine für eine Benutzung im Gehirn bestimmte intrazerebrale Elektrode (1), wobei diese Elektrode (1) die Form eines schmalen zylindrischen und länglichen Stifts mit zumindest einer elektrischen Kontaktfläche (2, 4) aufweist, die dazu bestimmt ist, mit einem elektrischen Leiter verbunden zu werden, der an ein Verarbeitungs- oder Aufnahmegerät angeschlossen wird, bei dem besagter Stift ein aus einem biokompatiblen starren oder halbstarren Material hergestelltes zylindrisches Substrat (10) bildet, **dadurch gekennzeichnet, dass** um dieses herum mit einem physikalischen Dampfniederschlagsprozess zumindest eine Metallschicht durch eine Maske (12) hindurch abgeschieden wird, die ein Muster bestimmt, das ausgelegt ist, um zumindest besagte Kontaktfläche (2, 4) zu definieren.

9. Elektrode nach Anspruch 8, **dadurch gekennzeichnet, dass** besagte elektrische Kontaktfläche (2, 4) aus Gold hergestellt wird.

## Claims

1. Method for manufacturing an electrode for medical use, such as an intracerebral electrode (1) intended for use at brain level, this electrode having the shape of a narrow, cylindrical and elongated rod including at least one electrical contact pad (2, 4) intended to be connected to an electrical conductor intended to be connected to a processing and/or recording device, wherein to realize said electrode (1), one uses a substrate (10) having a cylindrical shape which defines the body of the electrode, made out of a biocompatible rigid or semi-rigid material, **characterized in that** one deposits a metal layer on the surface of said cylindrical substrate (10) by means of a physical vapor deposition technique through a mask (12) that determines a pattern arranged so as to define at least said electrical contact pad (2, 4).

2. Method according to claim 1, **characterized in that** said metal layer is deposited on the whole of the circumference of said substrate (10), **in that** said metal deposition is carried out in at least two successive steps and **in that** said substrate (10) is turned over between said two successive steps.

3. Method according to claim 1, **characterized in that** said metal layer is deposited on the whole of the circumference of said substrate (10) and **in that** said metal deposition is carried out in one single step using at least two targets.

4. Method according to claim 1, **characterized in that** said metal layer is deposited on the whole of the circumference of said substrate (10) and **in that** said metal deposition is carried out by subjecting said substrate (10) to a rotation.

5. Method according to any of the previous claims, **characterized in that** a stainless steel mask (12) that forms a gripper arranged to clamp said substrate (10) is used.

6. Method according to claim 1, **characterized in that**, prior to the deposition of the metal layer, the area of the substrate (10) that is not covered by said mask (12) is activated chemically by means of an ionic cleaning carried out by means of a mix of oxygen and argon in the plasma state, and a layer of titanium is then deposited on it.

7. Method according to claim 1, **characterized in that** the metal used for defining said electrical contact pad (2, 4) is gold.

8. Electrode for medical use, obtained by the implementation of the method according to any of claims 1 to 7, such as an intracerebral electrode (1) intended for use at brain level, this electrode (1) having the shape of a narrow, cylindrical and elongated rod including at least one electrical contact pad (2, 4) intended to be connected to an electrical conductor intended to be connected to a processing and/or recording device, wherein said rod forms a cylindrical substrate (10) made out of a biocompatible rigid or semi-rigid material, **characterized in that** around which at least one metal layer is deposited by means of a physical vapor deposition technique through a mask (12) that determines a pattern arranged so as to define at least said electrical contact pad (2, 4).

9. Electrode according to claim 8, **characterized in that** said electrical contact pad (2, 4) is made out of gold.
